# EUROPEAN PATENT APPLICATION

(11) **EP 1 801 593 A1**
(43) Date of publication of application: **27.06.2007**
(21) Application number: 05112781.9
(22) Date of filing: 22.12.2005
(51) Int. Cl.: G01N 33/58

(54) **A method of imaging biological specimens using inorganic nanoparticles as label agents**

(71) Applicant: Koninklijke Philips Electronics N.V., 5621 BA Eindhoven (NL); FEI COMPANY, Hillsboro, Oregon 97124-5793 (US)
(72) Inventor: The designation of the inventor has not yet been filed
(74) Representative: Schmitz, Herman Jan Renier

(57) **Abstract**

This invention relates to a method of imaging biological specimens using inorganic luminescent nanoparticles as label agents. The nanoparticles comprise a host lattice doped with luminescent activator ions, where the activator ions serve as luminescent centers for the nanoparticle. The imaging is performed using light microscopy, electron microscopy, correlative (light/electron) microscopy and/or X-ray microscopy.

## Description

### FIELD OF THE INVENTION

The invention relates to a method of imaging biological specimens using inorganic luminescent nanoparticles as label agents comprising a host lattice doped with luminescent activator ions. Also, the invention relates to said inorganic nanoparticle with a functionalized surface and the use of said nanoparticle as label agent in imaging biological specimens. Further the invention relates to a method for obtaining a 3D reconstruction of a sample containing inorganic luminescent nanoparticles.

### BACKGROUND OF THE INVENTION

Understanding the structural organization of biological materials, e.g. cells, is an essential prerequisite for understanding how cells function. First of all, this requires a powerful imaging technique to visualize the organization of cells at adequate resolution. Furthermore, since the cells are colorless, translucent and not very dense, the contrast of the imaging often needs to be enhanced by means of e.g. stains or labels. Additionally, the development of specific labeling allows localization and imaging of individual cellular features or macromolecules. The most common technique for imaging biological samples is light microscopy (LM) with resolution in the range 200 - 500 nm enabling imaging cells and organelles¹. On the other hand, cryo-transmission electron microscopy (cryo-TEM) imaging gives the possibility of imaging proteins with resolution of 1-5 nm and resolving their structure at an atomic level. Indeed it provides high resolution, but this is a very expensive and complicated analysis. Thus, there is a large gap between both techniques in the range of 5 - 200 nm, which is very important for a range of biological investigations. Providing a good imaging technique in this range would greatly help to image proteins and answer the questions: where in the cell are the proteins; how are they related; how can their location be linked to their function? The development of a new imaging technique, which can combine high resolution (in the nm range) with simple sample preparation and handling is of a high importance for life science research. A possible approach is the combination of LM with electron microscopy (EM) - i.e. development of correlative (or multi-scale) microscopy. Correlative microscopy allows mapping of proteins with LM and higher resolution localization at ultra-structural level with EM. Another possibility is applying X-ray microscopy. X-ray microscopy gives information complementary to optical and electron microscopy. In particular, in the 'water window' spectral region between the K-shell x-ray absorption edges of carbon (~ 290 eV) and oxygen (~540 eV), organic materials show strong absorption and phase contrast, while water is non-absorbing. This enables imaging of specimens several µm thick with high intrinsic contrast using x-rays with a wavelength of 2 - 4 nm. Soft x-ray microscopy can deliver 20 - 30 nm resolution images of hydrated cells up to ~ 10 µm thickness².

However, development of novel imaging techniques requires application of suitable labels in order to enhance the contrast of the imaging as well as to achieve specific localization of cell structures. One approach would be the application of existing labels. State of the art labels for light microscopy imaging of biological samples are fluorescent probes (dyes)¹. This is a well-established technique with a large choice of fluorescent molecules and wide range of applications. The disadvantages, however, are photobleaching of the fluorescent molecules as well as high sensitivity to the environment leading to fluorescent quenching^{3;4}. The labels for electron microscopy imaging of biological specimens have to be electron dense in order to provide the necessary contrast. Gold nanoparticles (immunogold) are the most common labels based on their high-density contrast (which is a function of the atomic number of the element). Several ways of combining the state of the art labels have been reported so far, including⁵:
- sequential labeling with gold and fluorescent-labeled antibodies that bind to the same marker;
- silver-enhancement of nanogold particles;
- use of autofluorescent protein antibodies;
- fluorescence photo-oxidation of diamino-benzidine⁶;

All these methods however could be unreliable and require multiple steps. Moreover in cases where multiple labeling is required, those methods are restricted to single or double labeling.

In addition, multiple labeling using CdSe/ZnS quantum-dot nanoparticles has been reported to be very promising for correlative microscopy studies of proteins⁵. Quantum dots were applied for multiple labeling using light, electron and correlative microscopy in rat cells and mouse tissue. Although semiconductor quantum dots are very promising, they have certain disadvantages. The main disadvantage of Cd, Pb, Se, Te, Hg - containing quantum dots is their citotoxicity⁷, which restricts the number of applications (especially for in vivo imaging). In addition, quantum dots have some limitations in case of multiple labeling. Small variations in the size of the same material dots result in large variations of the emission wavelengths. E.g. changing nanoparticle size from 1.9 nm to 5.2 nm in case of CdSe/ZnS results in emission maximum variations from 470 nm to 610 nm. More importantly, varying the particle size from 2.1 nm to 2.4 nm to 2.6 nm corresponds to emission wavelength shift from 505 nm to 525 nm to 545 nm, respectively. Whereas these three different emission wavelengths can be easily discriminated in light fluorescent microscopy, the discrimination of 0.3 or even 0.5 nm size differences in TEM would be quite difficult and would require high resolution (HR) TEM mode. For biological specimens however, the dose should be kept as low as possible to minimize the damage on the specimen. In this way applying HR TEM is not desirable for discriminating the multiple labels. Thus, development of new types of labels, with desirable properties (such as stability and robustness under electromagnetic irradiation, suitable excitation and emission lines/bands of the luminescence, absence of bleaching, high luminescence quantum efficiency, strong scattering or/and diffraction of electromagnetic radiation or particle beam) is required. It is desirable to provide a single stable label material, suitable for application in light microscopy, electron microscopy, correlative microscopy and X-ray microscopy and providing good contrast of the imaging for all of the imaging techniques.

### SUMMARY OF THE INVENTION

The object of the present invention is to improve prior art methods of imaging biological specimens by means of using label material, which provides efficient contrast in microscopy imaging of biological specimens.

According to one aspect, the present invention relates to a method of imaging biological specimen comprising:
- using inorganic luminescent nanoparticles as label agents, said nanoparticles comprising a host lattice doped with luminescent activator ions acting as a luminescent centers for said nanoparticles,
wherein said imaging comprises activating said activator ions by an electromagnetic radiation source or particle beam and detecting the light emitted from said activator ions.

The emitted light from said activator ions, and thereby from said nanoparticles, will result in an increase in the contrast of the microscopy imaging and/or for detecting the presence of a detectable substance in a material. One of the many advantages obtained by using said nanoparticles as label agents is that they are stable and robust under broad range of excitation sources including light, electron beam, X-rays or combination of the above. Also, the nanoparticles are, contrary to fluorescent molecules showing photobleaching, highly photo-stable as well as stable and robust under e.g. electron beam or X-ray excitation. Additionally, the nanoparticles are very bright emitters showing high luminescent quantum yields, which improve the signal to noise ratio. Another advantage obtained by using said activator ions is related to the sharp emission lines from said activator ions. The result is a much better signal-to-noise ratio compared to band emitters such as organic fluorescent labels or quantum dots. Additional advantage is that the activator ions with sharp emission lines are very suitable for multiple labeling and detection, in contrast to the fluorescent dyes where broad emission bands overlap and restrict multiple labeling. Still another advantage obtained by using said nanoparticles doped with activator ions as labels is that the wavelength of the emitted light does, contrary to semiconductor quantum dots, not depend on the size and/or shape variations of the nanoparticles but only on the type of activator ions used. Thereby, changing the type of activator ions results in a change of the emitted wavelength, which makes the nanoparticles suitable for multiple labeling and discrimination of the labels, based on difference in the emission colors. An example of applications implementing said nanoparticles as label agents is e.g. in fixed cell imaging, cell tracking, imaging of whole cells, organelles, tissue sections; immunochemistry, multiplex fluorescent in situ hybridisation (FISH), for ex vivo cell imaging - imaging of membrane surface receptor, intracellular organs and molecules, and for biosensor applications. In addition, in vivo imaging in light microscopy combined with post-fixation and imaging in SEM and or TEM.

By the term nanoparticle in the present invention is meant a particle in the range of approximately 1nm-1000nm, but preferably in the range 1nm-100nm. However, under certain applications, the dimension could be under 1nm and/or above 1000nm.

In an embodiment, the surface of said nanoparticle is functionalized wherein the functionalization is obtained by solubilisation ligands attached to the nanoparticles surface, coating the nanoparticles surface, functionalization ligands attached to the nanoparticles surface containing at least one linking agent having a first portion linked to the nanoparticle and a second portion capable of linking to an affinity molecule, or combination of the above.

Surface functionalization of the nanoparticles has multiple effects. First of all, passivation of the nanoparticle surface is obtained, which prevents the excitation energy from non-radiative losses and thereby enhances the luminescence quantum efficiency from the nanoparticle. The result is an improved signal to noise ratio and improved detection during imaging. Also, nanoparticles with functionalized surface form stable water suspensions/solutions without aggregation of the nanoparticles. This improves sample preparation procedure and enhances the labeling process of the biological materials. More important, surface functionalization allows attachment of linking agents to the nanoparticle surface. In this way the one end of the linking agent is attached to the nanoparticle and the second end is attached to an affinity molecule.

In an embodiment, said luminescent activator ions are selected from a group consisting of rare earth ions, transition metal ions, ions with d¹⁰ and s² configuration, as well as d⁰ complex ions. Preferably the activator ions are selected from the rare earth ions or transition metal ions. The resulting luminescence from the nanoparticles will therefore be in the UV, visible or NIR. A particular advantage by using e.g. rare earth and/or transition metal ions as luminescent centers is that the luminescent lifetime of the nanoparticles becomes higher compared to prior art labels. This has the advantageous effect that the problem associated to the auto-fluorescence for the specimen will be avoided. The decay time of the auto-fluorescence of many biological specimens is in the ns range, while the decay time of said rare earth and transition metal ions is in the µs or even ms range. Accordingly, using said rare earth and/or transition metal ions as activator ions, the luminescent lifetime of the nanoparticle would be longer than that from the biological specimen. This will give a better signal-to-noise ratio compared to band emitters such as organic fluorescent labels and enhances the resolution of the imaging. An additional advantage obtained by using said activator ions is related to the sharp emission lines from said activator ions, which allow multiple labeling and detection, which is in contrast to the fluorescent dyes where broad emission bands overlap and restrict multiple labeling. The result is a much better signal-to-noise ratio compared to band emitters such as organic fluorescent labels or quantum dots.

In an embodiment, said inorganic luminescent nanoparticles comprise host lattices doped with a mix of two or more different types of activator ions, respectively. It follows that under certain type of radiation one type of the ion can act as an energy absorber, whereas the other type of ions acts as an emitter. An example of such scenario is LaPO₄:Ce³⁺,Tb³⁺, where under UV excitation the energy is absorbed by the Ce³⁺ and transferred to Tb³⁺ which emits afterwards visible light.

In an embodiment, said inorganic luminescent nanoparticles comprise host lattices doped with different types of activator ions, respectively. As mentioned previously, the type of the activator ions determines the emission wavelength from each respective nanoparticle. Thereby nanoparticles having the same types of host lattices can generate different emission colors by varying the types of the activator ions in the host lattices. Accordingly, multiple labeling can be achieved by implementing nanoparticle labels doped with various types of activator ions resulting in an emission of different characteristic colors.

In an embodiment, said host lattices are selected from a group consisting of insulators including metal salts or oxides and semiconductors. Thereby, a large variety of host lattices are provided for hosting said activator ions. However, a particular advantage obtained using insulators including metal salts or oxides as host lattices or non-heavy metal semiconductor materials is due to their low toxicity which is an advantage for certain biological applications such as in vivo imaging.

In an embodiment, said inorganic luminescent nanoparticles comprise nanoparticles which differ in their shape, their size, or a combination thereof, and wherein the discrimination of said nanoparticles is based on said differences. These variations in the sizes and/or shapes of the particles can be distinguishing in electron microscopy (e.g. TEM or STEM) or X-ray microscopy and therefore be applied for multiple labeling in electron microscopy or X-ray microscopy. In a preferred embodiment, said nanoparticles comprise a combination of nanorods and nanospheres. This is of particular advantage since the nanorods are easily distinguished from the nanospheres.

In an embodiment, said inorganic luminescent nanoparticles comprise host lattices of different elemental composition and wherein the discrimination of said nanoparticles is based on said differences in the elemental composition. Therefore, said difference in the elemental composition of the nanoparticles can be used for discrimination between the different nanoparticle labels and therefore be applied for multiple labeling.

In an embodiment, said method further comprises detecting backscattered electrons, secondary electrons, transmitted electrons, or a combination thereof from said inorganic nanoparticles and/or said biological specimen. Thereby, the resolution of the imaging of the biological specimen will be greatly increased, i.e. from micro scale to nano/atomic scale due to the use of electron microscopy, correlative light and electron microscopy or X-ray microscopy. The resolution gap between prior art methods, which implement light microscopy, or electron microscopy is thereby strongly reduced, or even eliminated. It is very advantageous that the nanoparticles are suitable labels for all microscopy techniques, since it will reduce the specimen preparation steps and simplify the preparation procedure. Moreover, the dynamics of reactions can be followed and it will allow the location of proteins to be linked to their function.

In an embodiment, detecting said emitted light and said backscattered electrons, secondary electrons, transmitted electrons or a combination thereof from said inorganic nanoparticles and/or said biological specimen is performed in a microscope using light, X-rays, a beam of electrons, a beam of ions, or any combination thereof.

According to another aspect, the present invention relates to the use of an inorganic luminescent nanoparticles as label agents applied in imaging biological specimens, said nanoparticles comprising a host lattice doped with luminescent activator ions, wherein said ions serve as luminescent centers for said nanoparticles.

In an embodiment said use is in light microscopy, electron microscopy, X-ray microscopy, or a combination of light microscopy and electron or X-ray microscopy. By combining said imaging techniques, the resolution of the imaging will be greatly enhanced.

According to still another aspect the present invention relates to an inorganic nanoparticle to be used as labeling agents in imaging biological specimen, comprising:
- a host lattice doped with luminescent activator ions, wherein said ions serve as luminescent centers for said nanoparticle, and
- a functionalized surface.

Thereby, a labeling agent is provided which is very stable and robust under broad range of excitation sources including light, electron beam, X-rays or combination of the above, wherein said labeling agent (nanoparticles) has multiple labeling property. As mentioned previously, the functionalization of the surface stabilizes the nanoparticles and prevents their agglomeration; helps to enhance the luminescence efficiency and most importantly provides biological functionalities such us specific labeling, membrane crossing, enzymatic functions etc.

In an embodiment said functionalized surface comprises:
- solubilization ligands attached to the nanoparticle surface,
- coating,
- functionalisation ligands attached to the nanoparticles surface, said ligands containing at least one linking agent having a first portion linked to the nanoparticle and a second portion capable of linking to an affinity molecule,
- ligands attached to the nanoparticles surface for creating membrane crossing or cell-internalization capability,
- ligands attached to the nanoparticle surface for creating enzymatic functions,
- combination of the above.

In an embodiment, said nanoparticle is conjugated/linked to an affinity molecule capable of bonding with a detectable substance, wherein the affinity molecule is selected from a group consisting of:
- a biological material,
- an antibody,
- a nucleic acid,
- a protein,
- a polysaccharide,
- a sugar,
- a peptide, and
- a drug.
   The affinity molecule is capable to specifically bond to a detectable substance in the biological material. In this way detecting the presence of the nanoparticle by detecting the luminescence from it or by detection based on density contrast in electron microscopy is an indication of the presence of this detectable substance in the biological material. As a result, the detectable substance can be localized and imaged with higher resolution. In case of multiple labeling two or more substances in the biological material (e.g. two or more different cell components or macromolecules) can be discriminated thanks to the multiple labels. In this way the dynamics of biochemical processes can be followed and imaged providing valuable information on different cell functions.
   According to yet another aspect of the invention a 3D reconstruction of a sample containing inorganic luminescent nanoparticles is obtained, the method comprising
- obtaining a plurality of images of the sample, and
- removing one of more surface layers from the sample between the moment that the first image is obtained and the moment that the last image is obtained.

Nanoparticles located at a certain depth in the sample will either not be excited or the light emitted by the nanoparticles will not be detected efficiently. By obtaining a plurality of images of the sample and while removing surface layers from the sample, a series of images is made that represent a 3D cross section of the sample. This method is especially effective when the nanoparticles are activated with e.g. electrons, as the range of electrons in a sample is very limited.

It is remarked that the removal of a surface layer need not be a uniform removal of the surface layer. It is expressly included that certain features on the surface of the sample, such as the inorganic nanoparticles, are not as easily removed as the surrounding material, such as biological tissue.

It is also remarked that the steps of obtaining an image and removing a surface layer can be subsequent steps, but that the steps can also overlap in time, for example by obtaining images while continuously removing the surface of the sample.

In an embodiment the one or more surface layers are removed by etching.

The etching of a sample by exposing the sample to e.g. certain gasses is a well-known process.

It is remarked that the etching can be assisted by irradiating the sample with e.g. light, electrons or ions, said irradiation e.g. forming the reactive gasses in the vicinity of the sample.

In another embodiment the one or more surface layers are removed by sublimation.

The removal of the surface layer by sublimation is especially suited when observing hydrated biological tissues.

In yet another embodiment the one or more surface layers are removed by sputtering.

The removal of a surface layer of a sample by sputtering the sample with an atomic beam or an ion beam is known as such. This sputtering can be assisted and/or accelerated by the admission of gasses.

In still another embodiment the removal of the one or more surface layer is performed in-situ in a particle-optical apparatus.

Particle-optical apparatus employing beams of electrons, ions or a combination of such beams are routinely used for the imaging of samples. For this use the samples are normally exposed to vacuum.

The electrons and/or ions can also be used to remove a surface layer, especially when gasses are admitted in the vicinity where the beams impinge onto the sample.

Also sublimation can be performed in such (evacuated) apparatus by controlling the temperature of the sample and/or the temperature of the environment of the sample.

By performing the imaging and the removal in-situ, no time is lost for the transport of the sample and the insertion of the sample in different apparatus, resulting in a time efficient process. Also the chance of damaging the sample due to transport and handling is minimized.

The aspects of the present invention may each be combined with any of the other aspects. These and other aspects of the invention will be apparent from and elucidated with reference to the embodiments described hereinafter.

### BRIEF DESCRIPTION OF THE DRAWINGS

Embodiments of the invention will be described, by way of example only, with reference to the drawings, in which
FIG. 1 illustrates graphically one embodiment of an inorganic luminescent nanoparticle according to the present invention,
FIG. 2 illustrated graphically another embodiment of an inorganic nanoparticle,
FIG. 3 shows a mix of nanoparticles comprising a combination of nanorods and nanospheres,
FIG. 4 illustrates the three steps of imaging process using nanoparticle label agents,
FIG. 5 shows a TEM image of LaPO4:Ce³⁺,Tb³⁺ spherical nanoparticles,
FIG. 6 shows a high-resolution TEM image of the same LaPO4:Ce³⁺,Tb³⁺ spherical nanoparticles from fig. 5,
FIGS. 7-8 show, respectively, TEM and High-resolution TEM images of LaP04:Ce³⁺,Tb³⁺ nanorods,
FIG. 9 shows EDX spectra acquired at randomly chosen location on the sample in FIGS. 7-8,
FIG. 10 shows the photoluminescence spectrum (λ_{exc} = 275 nm) of a dilute colloidal solution (0.02 wt%) of the LaPO4:Ce³⁺, Tb³⁺ spherical nanoparticles present in FIGS 5 and 6,
FIG. 11 shows cathodoluminescent spectrum of LaPO4:Ce³⁺, Tb³⁺ nanoparticles powder (nanorods from Figs. 7-8) excited using electron beam in a SEM where the emitted light was detected,
FIGS. 12and 13 show SEM images of the LaPO₄:Ce³⁺, Tb³⁺ nanoparticles detecting, respectively, secondary electrons (SE) and panchromatic cathodoluminescence (panCL) at the same position of the sample,
FIG. 14 shows BSE image of a LaPO₄:Ce³⁺, Tb³⁺ nanoparticle,
FIG. 15 shows HR SEM image (SE) ofa~5nm LaPO₄:Ce³⁺, Tb³⁺nanoparticle,
FIGS. 16 and 17 show TEM and HR TEM images of LaPO4:Eu³⁺ nanoparticles,
FIG. 18 shows a photoluminescence spectrum of a colloidal solution of LaPO₄:Eu (λ_{exc} = 260 nm) nanoparticles,
FIG. 19a and 19 b show TEM micrographs of oval (a) and spherical (b) YV04 nanoparticles,
FIG. 19c and 19d show high-resolution TEM micrographs showing oval (c) and spherical (d) YVO₄:EU nanoparticles, shown in Figs. 19(a) and (b) respectively, where the lattice fringes in the HR images can be discerned,
FIG. 20 shows the photoluminescent spectrum of the YVO₄:Eu³⁺ nanoparticles,
FIG. 21 shows photoluminescent spectra of YV04 nanoparticles doped with samarium and dysprosium, respectively,
FIG: 22 shows a SEM image of ZnS:Ag+,Cl nanoparticles synthesized with a polyol method,
FIG. 23 shows a SEM image of Y₂O₃:Eu³⁺ nanoparticles synthesized with a polyol method,
FIG. 24 shows photoluminescent spectra of ZnS:Ag⁺,Cl⁻ (λₑₓₜ = 366 nm) and Y₂O₃:Eu³⁺ (λₑₓₜ = 254 nm) nanoparticles synthesized using polyol method,
FIG. 25a-d shows TEM images of rare earth fluoride nanoparticles: NaYF₄ (10.5 ± 0.7 nm) (Fig. 25a), YF₃ nanorods (Fig. 25b), LaF₃ (8.0 ± 0.3 nm) (Fig. 25c), YbF₃(9.5 ± 0.6 nm) (Fig. 25d), and
FIG. 26 shows green up-conversion photoluminescent spectrum of Yb/Er co-doped NaYF₄ nanoparticles, excited with a 980nm laser.

### DESCRIPTION OF EMBODIMENTS

The present invention relates to using inorganic luminescent nanoparticles as label/contrast agents applied in imaging biological specimens using e.g. light microscopy, electron microscopy, correlative light and electron microscopy or X-ray microscopy, where the particles are excited using light, electron beam, X-rays etc.

The nanoparticle labels consist of an inorganic nanoparticle preferably with functionalized surface suitable for biological imaging or an inorganic nanoparticle with functionalized surface preferably containing at least one linking agent linked to an affinity molecule capable of bonding to a detectable substance. In this way detecting the light emitted from the label or detecting the presence of the label based on SEM imaging or TEM imaging or STEM imaging is an indication of the presence of the detectable substance in the biological material.

The nanoparticles comprise host lattices doped with activator ions, preferably rare earth ions or transition metal ions, or ions with d¹⁰ and s² configuration, as well as d⁰ complex ions, where the ions are used as luminescence centers for the nanoparticles. The type of the activator ions that determine the emission colors from the labels, generate this luminescence in the UV, visible or NIR. In addition to the luminescence, the presence of the nanoparticle labels in the investigated material (specimen) can be detected by detecting backscattered electrons (BSE) and/or the secondary electrons (SE) and/or the transmitted electrons from the material.

Accordingly, said nanoparticles may be used for multiple labeling simply by e.g. using different types of activator ions, thereby varying the emission colors from the nanoparticles. Such color differences are easily detectable. Also, such a multiple labeling is obtained by varying the sizes and shapes of the inorganic luminescent nanoparticles, since such variations will be detected in e.g. electron microscopy (TEM or STEM). Additionally, multi labeling can be obtained by varying the type of host material since different materials can be easily discriminated by use of EDS or EELS in SEM or TEM based on different elemental composition. Said multi labeling will be discussed in more details later.

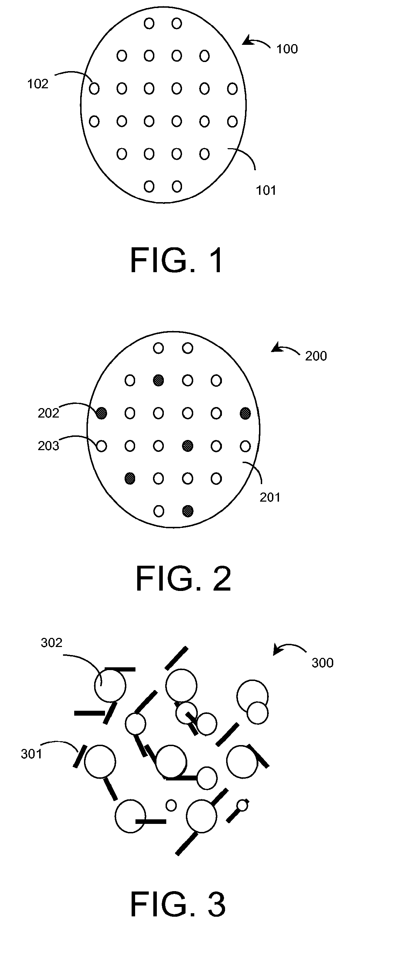

Figures 1 illustrates graphically one embodiment of an inorganic luminescent nanoparticle 100 to be used as a label agent in imaging biological specimens. Said nanoparticle comprises a host lattice 101, which can comprise insulating material, e.g. metal salt or oxide, or a semiconductor, doped with activator ions 102.

In one preferred embodiment, the activator ions 102 are made of luminescent rare earth ions with line emission (f-f), e.g. Eu³⁺, Tb³⁺, Sm³⁺, Dy³⁺, Pr³⁺, Nd³⁺, Tm³⁺, or rare earth ions with band emission - Ce³⁺, Pr³⁺, Eu²⁺, Sm²⁺. In another preferred embodiment, the activator ions 101 comprise transition metal ions, e.g. Cr³⁺, Mn⁴⁺, Mn²⁺. In all these nanoparticles the dopant ions act as luminescent centers. Under excitation using electromagnetic radiation or particle beam (such as light, electron beam, x-rays, etc.), the dopant ion absorbs the excitation energy and emits light. As a result the characteristic emission of the luminescent center is observed (e.g. f - f transitions or 4f-5d transitions in case of the rare earth ions or d-d transitions for d-ions). The optical and spectroscopic properties of the same materials have been found to be independent on the preparational method as well as on the size and shape of the nanoparticles, but only depending on the activator ions (dopants). The same host lattice can be doped with different activator ions, resulting in different and variable emission colors (independent on the particle size and shape). E.g. LaPO₄ :Eu³⁺ emits in the red, while LaPO₄:Ce³⁺,Tb³⁺ is a green emitter.

Figure 2 illustrated graphically another embodiment of an inorganic nanoparticle 200, to be used as a label agent in imaging biological specimens comprising a host lattice 201 of a type as mentioned under Fig. 1, and two or more activator ions 202, 203 comprised within the same host. In this way one of the ions can act as a sensitizer and the second ion as an emitter.

Figure 3 shows a mix of nanoparticles 300 to be used as label agents in imaging biological specimens comprising a combination of nanorods 301 and nanospheres 302, doped with said activator ions, which may further comprise different sizes as shown. This is of particular advantage since the different variations in size and/or shape of the nanoparticles are easily detectable in electron and/or X-ray microscopy.

Although figures 1-3 illustrate the inorganic nanoparticles 100, 200, 300 as being a nanospheres or nanorods, their dimension and the symmetry can be different, e.g. pyramidal, cube-like, any kind of polyhedron, rod-like or simply be irregular. In general, the size and shape of the nanoparticles depends on the methods of their synthesis. Preparation methods for nano-phosphors and pigments reported in literature include solvothermal and hydrothermal synthesis¹³⁻¹⁹, polyol synthesis^{20;21}, precipitation²², sol-gel methods²³⁻²⁵, combustion synthesis²⁶, among others, resulting in well separated nanoparticles that can be re-dissolved in appropriate solvents giving rise to transparent and scatter-free colloidal solutions. Recent advances in synthetic methods for nanoparticles allow preparation of a large number of materials on the nanoscale with well-controlled size and shape as well as narrow size distribution. E.g. luminescent oxides (Y₂O₃), vanadates (YVO₄), phosphates (LaPO₄, CePO₄, YPO₄, etc), fluorides, etc have been synthesized. In some cases the same host lattice can form different types of nanoparticles - e.g. YVO₄ and LaPO₄ nanospheres and nanorods, as described in the examples below. The described particles can, as mentioned previously, be doped with different activator ions resulting in variable emission wavelengths.

The quantum efficiency (which in case of photoluminescence is defined as the ratio of the number of photons emitted by the nanostructure and the number of photons hitting the nanostructure) of said nanoparticles 100, 200, 300 can be enhanced by a surface functionalization (not shown in the figures) of said nanoparticles. As mentioned previously, such surface functionalization provides solubility and prevents aggregation in the desired media. Moreover it allows attachment of linking agents (e.g. functional groups such as - COOH or NH3) for bio-conjugation. The surface of the nanoparticles can be chemically modified by:
i) Using solubilization/functionalization ligands (i.e. complexing agents or surfactant molecules) attached to the nanoparticle surface:
   - Using dioles - e.g. heating the nanoparticles in ethylene glycol or other polyethylene glycols with various chain lengths.
   - Binding phosphonic acid or phosphonic acid ester derivatives, e.g. imino-bis(methylenphosphono) carbonic acid which could be synthesized using Mannich-Moedritzer reaction⁸. The phosphonic acid derivative should replace the complexing agent surrounding the particle surface. The displacement reaction is enhanced at higher temperature.
   - Organic acids, e.g. amino acids, which tend to bind to rare earth ions via their carboxylic groups- e.g. glycine⁹.
   - Non-covalent linking using chain-type molecules having a polarity or charge opposite to that of the nanoparticles - e.g. surfactants, acid or basic proteins, polyamines, polyamides, polysulfone or polycarboxlic acid.
ii) Applying coatings:
   - Applying silica coating around the nanoparticles. Silica allows a simple chemical conjugation of organic molecules since it reacts easily with organic linkers such as triethoxysilane or chlorosilane.
   - Applying polymer coating around the nanoparticles. Polymerisable complex agent are e.g. diacetilene, styrene butadiene, vinylacetate, acrylate, acrylamide, vinyl compounds, boron oxide, N-(3-aminopropil)-3mercaptobenzamidine, 3-(trimetoxysilyl)propylhydrazide, etc.
   - Bio-conjugation includes linking an affinity molecule (a bio-molecule) to the nanoparticle surface in order to introduce specific functionalities¹⁰ such as - specific labeling, membrane crossing, cell internalization etc. In case of specific labeling an affinity molecule, e.g. an antibody, attached to the nanoparticle binds specifically to a detectable substance in a biological material, e.g. to specific receptors. Bio-conjugation can be achieved by:

   - Absorbing the biomolecule on the nanoparticle surface, by replacing some of the surfactant molecules, e.g. partial ligand exchange;
   - Biomolecules can be attached non-specifically to the surfactant molecules on the nanoparticle surface using e.g. electrostatic interactions;
   - Biomolecules can be covalently bond to the functional groups (e.g. -COOH or NH3) of the surfactant molecules on the nanoparticles surface. Standard methods for bio-conjugation described in literature could be applied¹¹.
   - Instead of directly conjugating the biomolecule to the outer shell of the nanoparticle, they can be attached using steptavidin - biotin system as described in case of colloidal quantum dots¹⁰. For this purpose steptavidin is covalently bond to the nanoparticle surface and this particle is reactive to biomolecules containing biotin moiety.

In addition bio-conjugation can provide the nanoparticle -labels with membrane crossing or cell-internalization capability or enzymatic function¹². A large number of potential surface attachment groups could be used for a single nanoparticle, resulting in multifunctional coating of the nanoparticle.

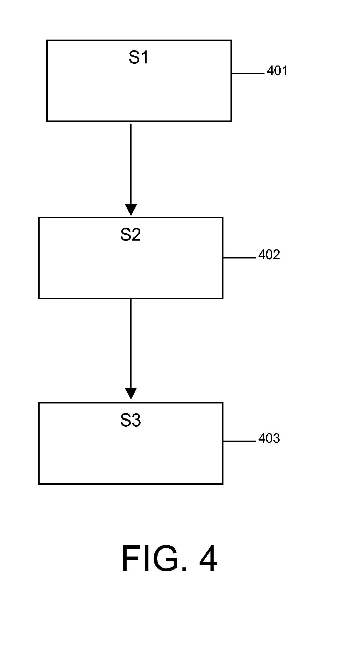

Figure 4 illustrates method steps of imaging biological specimen using nanoparticles shown in figures 1-3 as label agents.

The first step (S1) 401 is to activate the nanoparticle labels by an electromagnetic radiation source or particle beam, which can e.g. comprise X-rays, light, electron beam, or a combination thereof Under this excitation the energy is transferred to the dopant ions, which as mentioned previously, acts as a luminescence center for the nanoparticles. The result is a characteristic emission of the luminescent center (e.g. f - f transitions or 4f-5d transitions in case of the rare earth ions or d-d transitions for d-ions).

The second step (S2) 402 is detecting said emitted light from the nanoparticle label which is used to detect the presence of a specific substance in the material and to localize this substance during imaging in light microscopy, electron microscopy or X-ray microscopy. The detection of the emitted light can take place in a light microscope, an electron microscope including SEM, TEM and STEM, or an X-ray microscope.

In case of multiple labeling, e.g. labeling of two or more different substances in the biological materials, two or more different labels can be applied, each of them with different emission color. These different labels can be discriminated based on the differences in the emission color.

In addition, in case of multiple labeling and imaging in TEM or STEM, the different nanoparticle labels can, as mentioned previously, differ in their size and or shape. In this way, in addition to the different emission color, the labels can be discriminated based on their different size and/or shape.

In addition, multiple labeling can, as mentioned previously, be achieved by using two or more labels with different elemental composition. The discrimination of the labels can be achieved by detecting their elemental composition with EDS or EELS.

In the third step the labeled substance in the material can be localized and further imaged using light, electron or X-ray microscopy.

### Examples:

### Example 1:

### Luminescent phosphates^{17;18;30}: LaPO₄:Ce³⁺,Tb³⁺ and LaPO₄:Eu³⁺

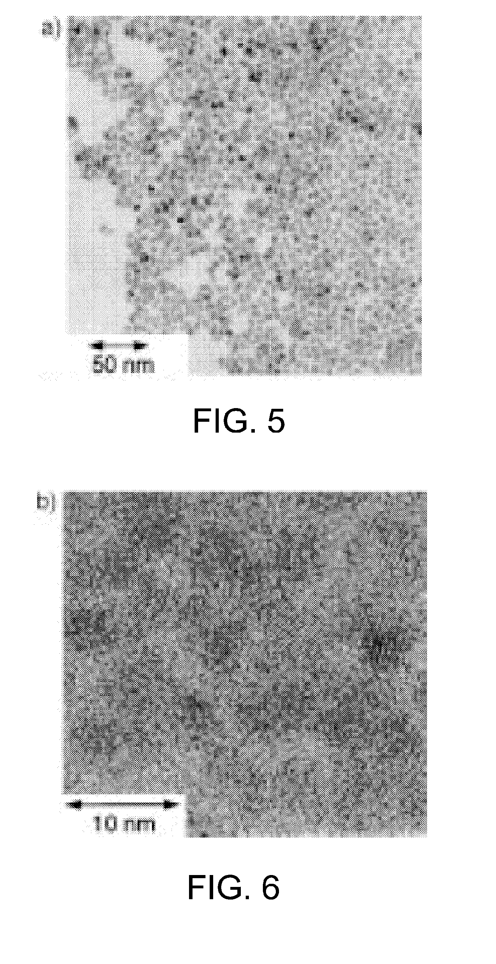

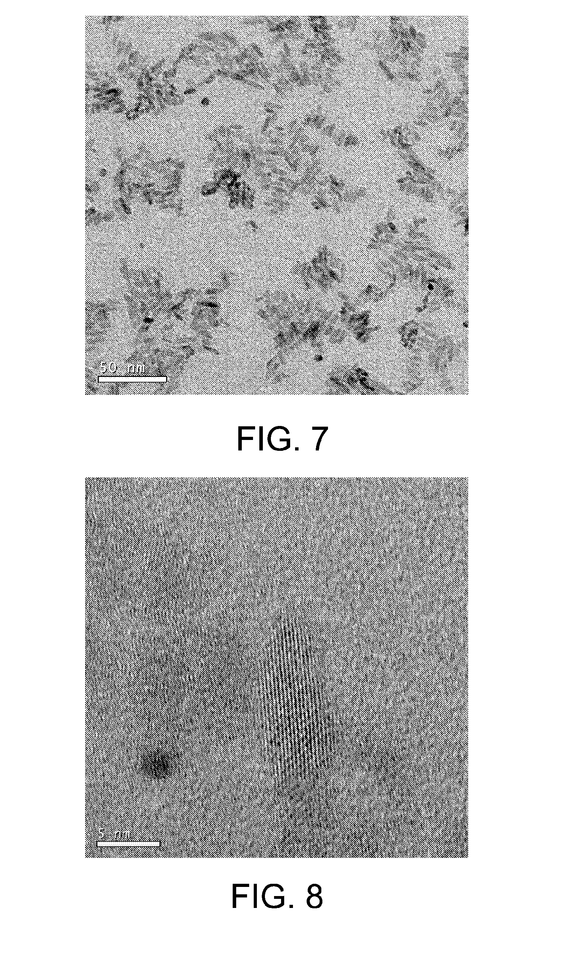

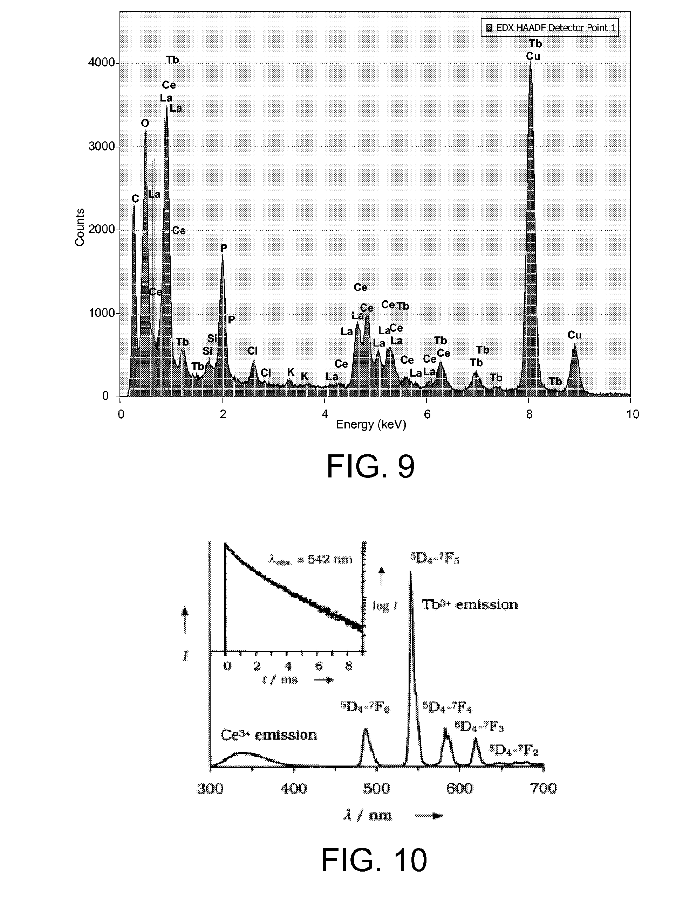

LaPO₄:Ce³⁺,Tb³⁺ nanoparticles can be produced applying liquid-phase synthesis (in high-boiling coordinating solvents)¹⁸. Two types of nanoparticles can be obtained - spherical with average particle size of~5-6 nm or oval (nanorods) with length of ~ 15 -18 nm and width of ~ 4 - 5 nm. Figures 5 and 6 shown, respectively, TEM image (Fig. 5) and HR TEM image (Fig. 6) of LaPO4:Ce³⁺,Tb³⁺ spherical nanoparticles (images taken from ref.¹³). Figures 7-8 shown, respectively, TEM and High-resolution TEM image of LaP04:Ce³⁺,Tb³⁺ nanorods. Figures 5-8 demonstrate clearly that two different types of nanoparticles can be distinguished not only in HR TEM imaging, but also with conventional TEM, and thus applied for multiple labeling. In addition, EDX can be applied to analyze the elemental composition (Fig. 9). Figure 9 shows EDX spectra acquired at randomly chosen location on the sample. The signals of the expected elements like La, P, O, Ce and Tb can clearly be discerned. The Cu-signal is caused by the Cu-grid and the C- and Si-signals by the carbon foil (and C-contamination).

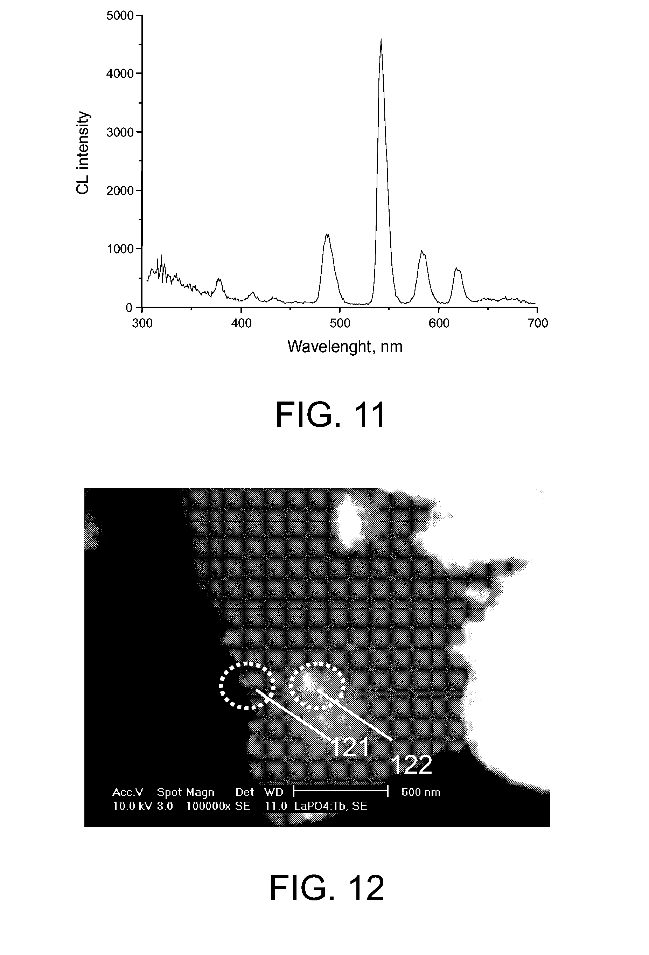

The luminescent spectra of both the nanospheres as well as the nanorods are dominated by a green emission with maximum at around 550nm due to the ⁵D₄ - ⁷F₅ transitions of the Tb³⁺ ion (line f-f emission). Figure 10 shows the photoluminescent spectrum (λ_{exc} = 275 nm) of a dilute colloidal solution of the spherical nanoparticles from Figs. 5 - 6. Inset: Luminescence decay curve of the same colloidal solution (λ_{exc} = 266 nm, τ_{exc} = 15 ns; detection wavelength λ_{obs} = 542 nm, image taken from ref.19). Figure 11 shows cathodoluminescent spectrum of the powder sample of LaP04:Ce³⁺,Tb³⁺ nanorods shown in Figs. 7 - 8.

The reported quantum efficiency of the Tb³⁺ emission is 42 % and the overall quantum efficiency of 61 % taking into account the Ce³⁺ emission at ~ 350 nm. (The quantum efficiency of the bulk material under 254 nm excitation is 87 % for the Tb³⁺ emission and 93 % for the total emission). The decreased quantum efficiencies of the nano-phosphors compare to bulk materials are due to the significantly increased surface-to-volume ratio responsible for non-radiative recombination at the particle surface and thus decreasing the quantum efficiency. However, additional coating and surface functionalization, as previously described, can be applied to reduce the non-radiative losses on the particle surface and enhance the quantum efficiency.

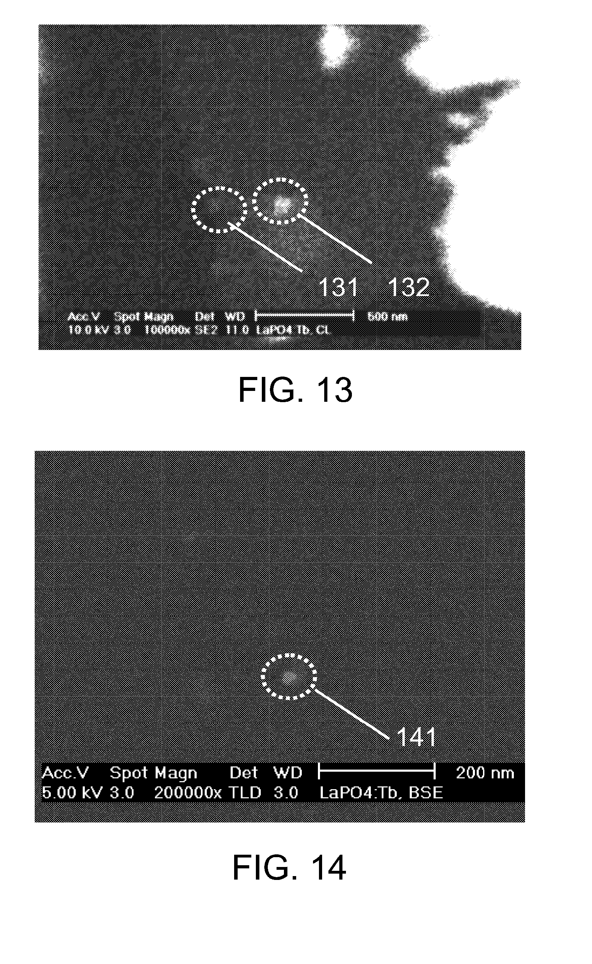

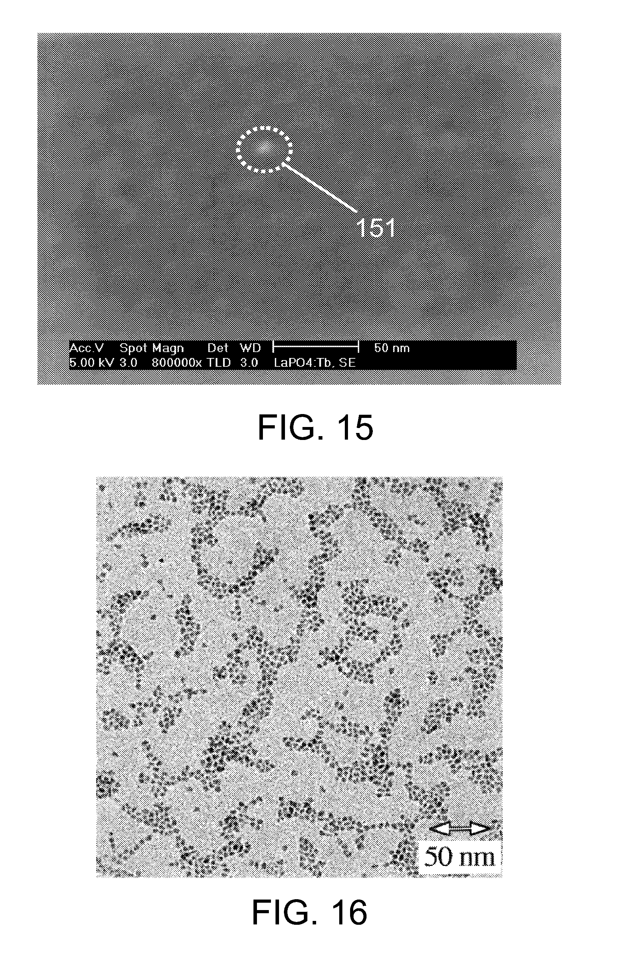

LaPO₄:Ce³⁺,Tb³⁺ nanoparticles are suitable to be applied as labels in electron microscopy or X-ray microscopy. They provide sufficient contrast when detecting secondary electrons (SE) and backscattered electrons (BSE) in a scanning electron microscope (SEM), as well as when detecting panchromatic cathodoluminescence (panCL). Figures 12 and 13 show SEM images of the LaPO₄:Ce³⁺, Tb³⁺ by detecting secondary electrons (SE) and panchromatic cathodoluminescence (panCL) at the same position of the sample. Both figures show a nanoparticle, 121, 131, of approximately 20 nm and an aggregate, 122, 132, of approximately 80 nm in the center of the images. Figure 14 shows BSE image of another ~ 20 nm particle 141. In high-resolution mode particles of~5 nm 151 could easily be detected (Figure 15).

In addition, for the time of the imaging no decrease in the luminescent intensity was noticeable, confirming the rigidity of the nanoparticles and their stability under electron beam irradiation.

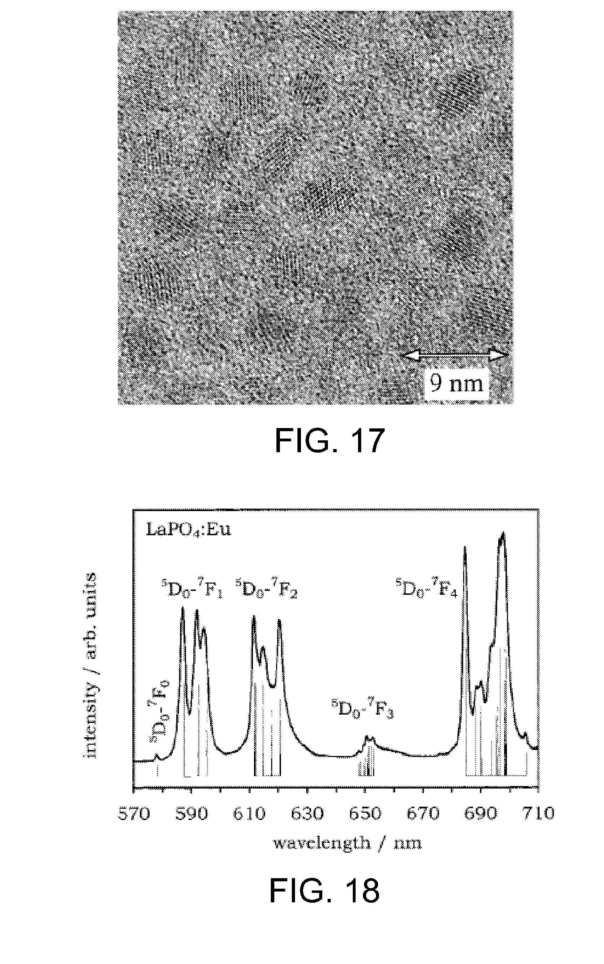

LaPO₄:Eu³⁺ nanoparticles with a mean particle size of 5 nm and a narrow size distribution were prepared by reacting the corresponding metal chlorides, phosphoric acid, and a base at 200 ⁰C in tris (ethylhexyl) phosphate (liquid-phase synthesis)³⁰. Figure 16 shows TEM and figure 17 a HR TEM image of LaPO₄:Eu³⁺ nanoparticles (images taken from ref.¹⁹). As the images show, the nanoparticles have spherical shape. The particles show emission in the red part of the spectrum. Figure 18 shows a photoluminescence spectrum of a colloidal solution of LaPO₄:Eu nanoparticles. Main lines of the emission spectrum are due to ⁵D₀ - ⁷F_{J} transitions of the Eu³⁺ ion (line f-f emission). Vertical lines indicate transitions from the ⁵D₀ level observed in bulk material (image taken from ref .15).

### Example 2:

### Luminescent vanadates¹⁹: YVO₄:Ln (Ln = Eu³⁺, Sm³⁺, Dy³⁺)

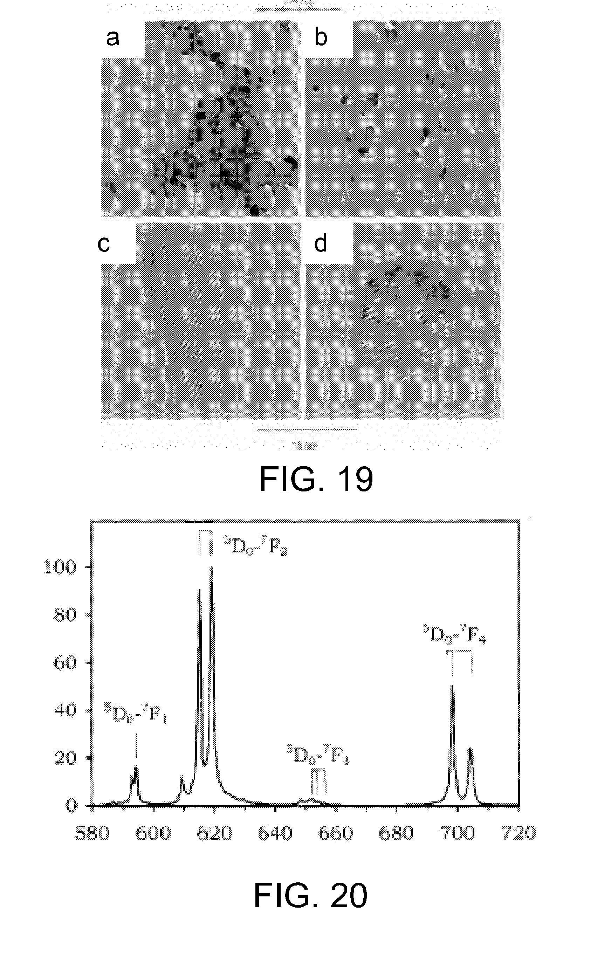

Colloidal solutions and redispersable powders of YVO₄:Ln nanoparticles were prepared using hydrothermal synthesis at 200 °C yielding nanoparticles with size between 10 and 30 nm. By varying the pH of the solution oval nanoparticles, in acidic solution ("method a") and spherical nanoparticles in strongly alkaline solution ("method b") were obtained. Figure 19a shows TEM micrographs of the oval nanoparticles of YVO₄:Eu, and figure 19b shows TEM micrographs of the spherical nanoparticles.

Figure 19c and 19d show high-resolution TEM micrographs showing lattice fringes of single YVO₄:EU nanoparticles prepared by "method a" (Fig. 19c) and "method b" (Fig. 19d). The images in figure 19 are taken from ref¹⁹.

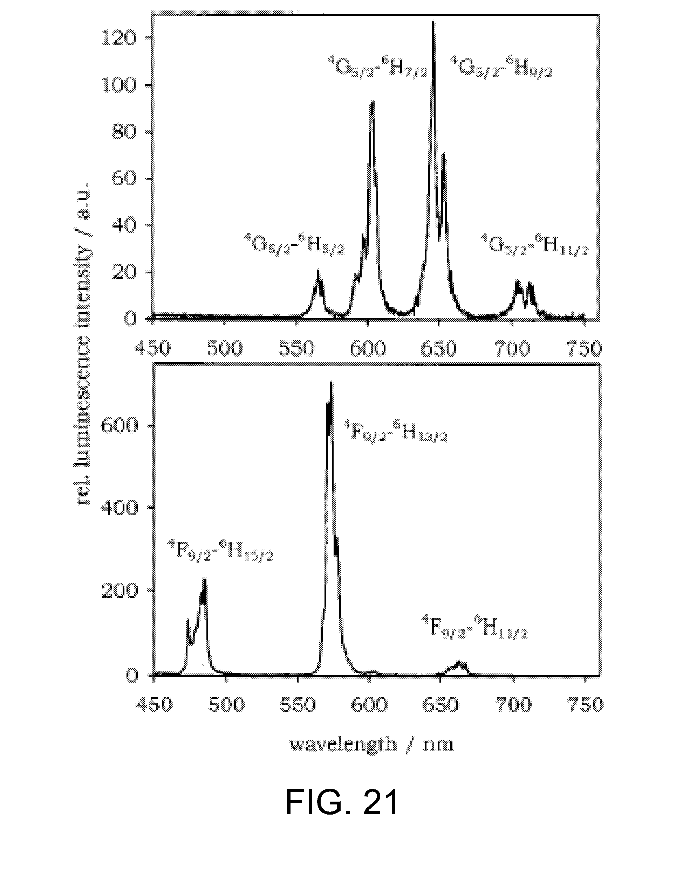

Figures 20 and 21 show the photoluminescent spectra of doped YVO₄ nanoparticles. Figure 20 shows the photoluminescence spectrum of a 4 x 10⁻⁴ M colloidal solution of YVO4:Eu³⁺ (λ_{exc} = 310 nm) nanoparticles. The main lines of the emission spectrum are due to transitions from the ⁵D₀ level of the Eu³⁺. Figure 21 shows photoluminescent spectra of YVO₄ nanoparticles doped with Sm³⁺ (top part) and Dy³⁺ (bottom part of Fig 21), respectively. The top part shows a luminescence of Sm³⁺ after excitation of the vanadate host (λ_{exc} = 310 nm), and the bottom part shows a luminescence of Dy³⁺ after excitation of the vanadate host (λ_{exc} = 310 nm). The images are from ref¹⁹.

The photoluminescent spectra of the doped YVO₄ nanoparticles were independent on the synthetic method or particle shape, showing characteristic emission of the lanthanide dopants (Figs. 20 and 21). The photoluminescent spectrum of YVO₄:Eu³⁺ shows Eu³⁺ f-f emission in the red dominated by the ⁵D₀ - ⁷F₁ transition at ~ 620 nm (Fig. 18). When doped with Sm³⁺ or Dy³⁺, YVO₄ emitted in the orange - red with emission maximums at 600 - 650 nm due to ⁴G_{5/2} - ⁶H transitions of the Sm³⁺ and at ~ 580 nm due to ⁴F_{9/2} - ⁶H transitions of the Dy³⁺, respectively.

### Example 3:

### ZnS:Ag⁺ nanoparticles:

### ZnS:Ag⁺ nanoparticles can be prepared using polyol-mediated synthesis²¹.

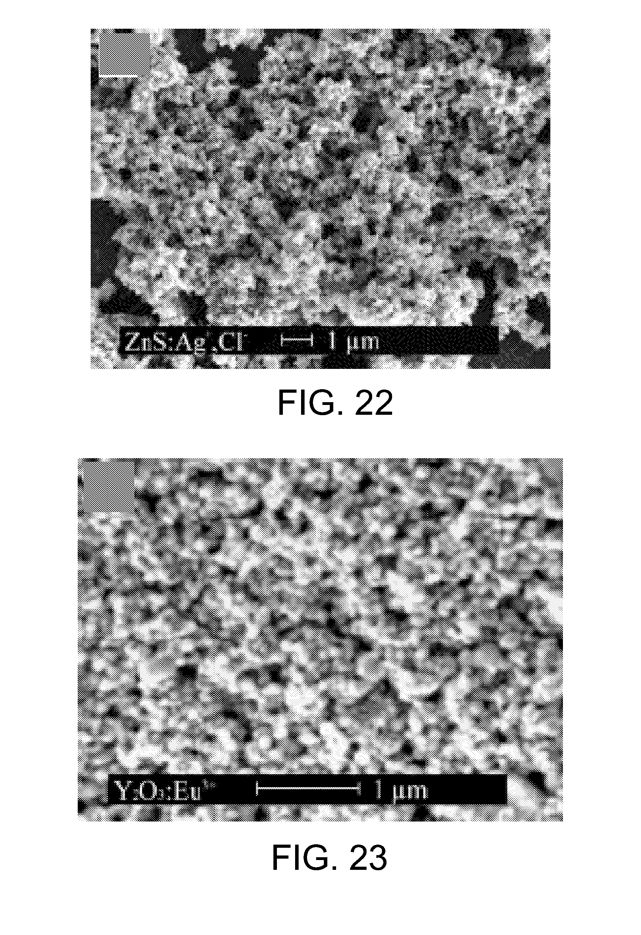

Figure 22 shows a SEM image of the ZnS:Ag⁺,Cl⁻ nanoparticles wherein the average particles size is 60 - 70 nm and the material is crystalline as prepared. ZnS:Ag⁺,Cl⁻ is a broad band emitter (donor-acceptor emission) with emission maximum at ~ 465 nm.

### Example 4:

### Y₂O₃:Eu³⁺ nanoparticles:

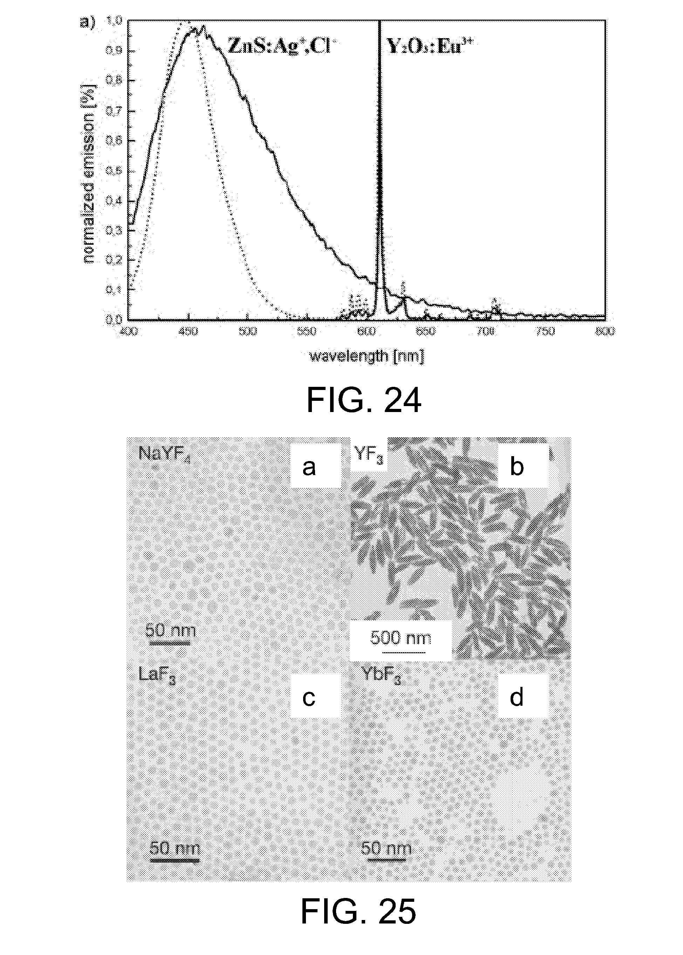

Applying polyol synthesi²¹, Y₂O₃:Eu³⁺ can be produced in a nanoscale with average particle size of 70-80 nm. Figure 23 shows a SEM image of the Y₂O₃:Eu³⁺ nanoparticle synthesized with a polyol method. Figure 24 shows the photoluminescent spectra of the ZnS:Ag⁺,Cl⁻ (λₑₓₜ = 366 nm) and the Y₂O₃:Eu³⁺ (λₑₓₜ = 254 nm) nanoparticles synthesized using polyol method. Photoluminescent spectra of the bulk materials are given as dotted lines. The line emission of the Y₂O₃:Eu³⁺ in red part of the spectrum is due to the ⁵D₀ - ⁷F_{J} transitions of the Eu³⁺ ion with the emission line at 613 nm dominating due to the hypersensitivity of the ⁵D₀ - ⁷F₂ transition.

### Example 5:

### Rare earth fluoride and hydroxide nanoparticles³¹.

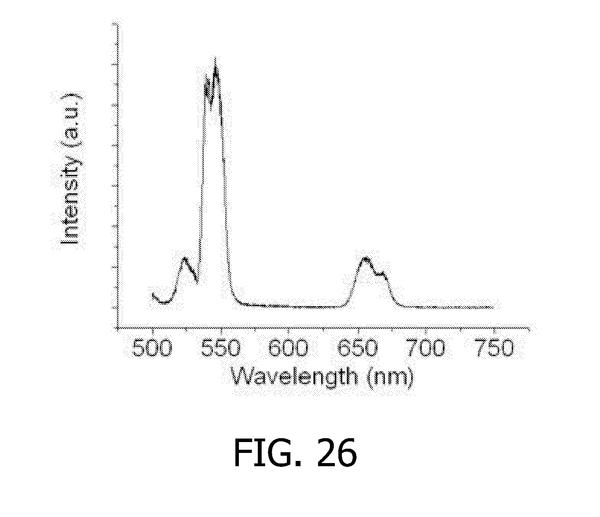

The synthesis of rare earth fluoride nanoparticles is based on a general phase transfer and separation mechanism occurring at the interfaces of the liquid, solid and solution phases present during the synthesis (LSS phase transfer)³¹. The LSS phase transfer and separation approach was used in generating nearly monodisperse rare earth fluorescent nanoparticles with up-conversion or down-conversion emission properties. Those nanoparticles with nano-spherical or nanorod shapes could be doped with different rare earth ions resulting in different emission wavelength. These nanoparticles could also be prepared by tuning the reaction at the interfaces of the different phases. Figure 25a-d shows TEM images of these rare earth fluorescence nanoparticles: NaYF₄ (10.5 ± 0.7 nm) (Fig. 25a), YF₃ nanorods (Fig. 25b), LaF₃ (8.0 ± 0.3 nm) (Fig. 25c), YbF₃(9.5 ± 0.6 nm) (Fig. 25d) (the images are taken form ref.³¹). As may be seen in Figs. 25a,c,d (NaYF₄, YbF₃ and LaF₃) the nanoparticles are approximately round, with a diameter in the range of 4 nm - 12 nm (that varied with temperature), whereas the nanorods YF₃ in Fig. 25b were characterized as having a rice-like shape with a diameter of ~100 nm and length of ~ 500 nm (composed of uniform nanoparticles with a diameter ~ 5 nm). Ln(OH)₃ products (images not shown) were usually composed of uniform nanorods with a diameter 3-15 nm (that varied with temperature). By doping different rare earth ions such as Gd³⁺, Eu³⁺, Nd³⁺, Tb³⁺ or Yb/Er these nanoparticles were functional as luminescent nanoparticles. Figure 26 shows green up-conversion emissions spectrum of Yb/Er co-doped NaYF₄ nanoparticles, excited with a 980nm laser. (The image is taken from ref.³¹).

Although the present invention has been described in connection with the specified embodiments, it is not intended to be limited to the specific form set forth herein. Rather, the scope of the present invention is limited only by the accompanying claims. In the claims, the term comprising does not exclude the presence of other elements or steps. Additionally, although individual features may be included in different claims, these may possibly be advantageously combined, and the inclusion in different claims does not imply that a combination of features is not feasible and/or advantageous. In addition, singular references do not exclude a plurality. Thus, references to "a", "an", "first", "second" etc. do not preclude a plurality. Furthermore, reference signs in the claims shall not be construed as limiting the scope.

### References:

1. Alberts and et al, in Molecular Biology of the Cell, (Garland Science, 2002) ,chap. 9.
2. Jacobsen C. Soft X-ray microscopy. Cell Biol. 9, 44-47. 1999.
3. Prodi L, New J.Chem. 29, 20-31 (2005).
4. Dulkeith E. and et al, Nano Letters 5, 585-589 (2005).
5. Giepmans B.N.G., Deerink, T. J., Smarr, B. L., Jones, Y. Z., and Ellisman M.H. Correlated light and electron microscopic imaging of multiple endogenous proteins using Quantum dots. Nature Methods 2, 743-749. 1-10-2005 and cited therein.
6. M. Grabenbauer and et al, Nature Methods 2, 857-862 (2005).
7. Kirchner C. and et al, Nano Letters 5, 331-338 (2005).
8. Moedritzer, J.Org.Chem. 31, 1603 (1966).
9. S. Wuister, C. Mello Donega, A. Meijerink, Phys.Chem.Chem.Phys. 6, 1633 (2004).
10. W. J. Parak, T. Pellegrino, C. Plank, Nanotechnology 16, R9-R25 (2005).
11. G. T. Hermanson, Bioconjugate Techniques (San Diego, CA: Academic Press, 1996).
12. X. Michalet et al., Science 307, 538-544 (2005).
13. Haase, M. and Riwotzki. Synthesis and properties of colloidal lanthanide-doped nanocrystals, J Alloys Comp. 191, 303-304. 2000.
14. Haase, M. G. S. Equiatomic rare earth (Ln) transition metal antimonides LnTSb (T=Rh, Ir) and bismuthides LnTBi (T=Rh, Ni, Pd, Pt). Journal of Solid State Chemistry.
15. M. G. S. Haase, H. Hoheisel, K. Bohmann, S. Haubold, K. Koempe, to be published (2005).
16. K. Koempe, H. H. Borchert, T. Moeller, M. G. S. Haase, Angew.Chem.Int.Ed. 42, 5513-5516 (2003).
17. Meyssamy, H., Riwotzki, K., Kornowski, A., Naused, S., and Haase, M. G. S. Wet-chemical synthesis of doped colloidal nanomaterials: particles and fibers of LaPO/sub 4/:Eu, LaPO/sub 4/:Ce, and LaPO/sub 4/:Ce,Tb. Advanced Materials 11, 840-843. 1999.
18. Riwotzki, H. Meyssamy, Schnablegger, A. Kornowski, M. Haase, Liquid-phase synthesis of colloids and redispersible powders of strongly luminescing LaP04:Ce,Tb nanocrystals, Angew.Chem.Int.Ed. 40, 573-576 (2001).
19. Riwotzki, K. and Haase, M. G. S. Colloidal YVO/sub 4/:Eu and YP/sub 0.95/V/sub 0.05/O/sub 4/:Eu nanoparticles: luminescence and energy transfer processes. J.Phys.Chem.B 102, 10129-10135 (1998).
20. Feldmann, C. Polyol mediated synthesis of oxide particle suspensions and their application. 5th International Conference on Nanostructured Materials (NANO 2000) 44(8-9), 2193-2196. 2001.
21. C. Feldmann, Polyol-Mediated Synthesis of Nanoscale Functional Materials, Advanced Functional Materials 13, 101-106 (2003).
22. A. Huidgnard, T. GAcoin, J.-P. Boilot, Chem.of Mater. 12, 1090 (2000).
23. Yin, M, Zhang, W., Xia, S., and Krupa J.-C. J.Lumin. 68, 335. 1-1-1996.
24. Goldburt E.T. and et al. J.Lumin. 75, 1. 1997.
25. Goldburt E.T. and et al. J.Appl.Phys. 83, 5404. 1998.
26. S. Wuister, C. Mello Donega, A. Meijerink, Phys.Chem.Chem.Phys. 6, 1633 (2004).
27. Medintz and et al, Nature Mater., 4, 435, 2005.
28. Michalet, X., Pinaud, F, Bentolila, L A, Tsay, J M, Doose, S, Li, J J, Sundaresan, G, Wu, A M, Gambhir, S S, and Weiss, S, Quantum Dots for Live Cells, in Vivo Imaging, and Diagnostics. Science, 307, 538, 2005.
29. Hermanson, G.T., Bioconjugate Techniques. 1996.
30. Riwotzki, H. Meyssamy, A. Kornowski, M. Haase, J.Phys.Chem.B 104, 2824-2828 (2000)
31. X. Wang and et al, Nature 437, 121-124 (2005).

## Claims

1. A method of imaging biological specimen comprising:
- using inorganic luminescent nanoparticles as label agents, said nanoparticles comprising a host lattice doped with luminescent activator ions acting as luminescent centers for said nanoparticles,
wherein said imaging comprises activating said activator ions by an electromagnetic radiation source or particle beam and detecting the light emitted from said activator ions.

2. A method according to claim 1, wherein the surface of said nanoparticle is functionalized wherein the functionalization is obtained by solubilisation ligands attached to the nanoparticles surface, coating the nanoparticles surface, functionalization ligands attached to the nanoparticles surface containing at least one linking agent having a first portion linked to the nanoparticle and a second portion capable of linking to an affinity molecule, or combination of the above.

3. A method according to claim 1, wherein said luminescent activator ions are selected from a group consisting of rare earth ions, transition metal ions, ions with d¹⁰ and s² configuration, as well as d⁰ complex ions.

4. method according to claim 1, wherein said inorganic luminescent nanoparticles comprise host lattices doped with a mix of two or more different types of activator ions, respectively.

5. A method according to claim 1, wherein said inorganic luminescent nanoparticles comprise host lattices doped with different types if activator ions, respectively.

6. A method according to claim 1, wherein said host lattices are selected from a group consisting of insulators including metal salts or oxides and semiconductors.

7. A method according to claim 1, wherein said inorganic luminescent nanoparticles comprise nanoparticles which differ in their shape, their size, or a combination thereof, and wherein the discrimination of said nanoparticles is based on said differences.

8. A method according to any of the claims 1-6, wherein said nanoparticles comprises a combination of nanorods and nanospheres.

9. A method according to any of the claims 1-8, wherein said inorganic luminescent nanoparticles comprise host lattices of different elemental composition and
wherein the discrimination of said nanoparticles is based on said differences in the elemental composition.

10. A method according to claim 1, wherein said method further comprises detecting backscattered electrons, secondary electrons, transmitted electrons or a combination thereof from said inorganic nanoparticles and/or said biological specimen.

11. A method according to claim 10, wherein detecting said emitted light and said backscattered electrons, secondary electrons, transmitted electrons or a combination thereof from said inorganic nanoparticles and/or said biological specimen is performed in a microscope using light, X-rays, a beam of electrons, a beam of ions or any combination thereof

12. A use of an inorganic luminescent nanoparticle as label agent applied in imaging biological specimens, said nanoparticles comprising a host lattice doped with luminescent activator ions, wherein said ions serve as luminescent centers for said nanoparticles.

13. Use according to claim 12 in light microscopy, electron microscopy, ion microscopy, X-ray microscopy, or any combination thereof.

14. An inorganic nanoparticle to be used as labeling agents in imaging biological specimen, comprising:
- a host lattice doped with luminescent activator ions, wherein said ions serve as luminescent centers for said nanoparticle, and
- a functionalized surface.

15. An inorganic nanoparticle according to claim 14, wherein said functionalized surface comprises:
- solubilization ligands attached to the nanoparticle surface,
- coating,
- functionalization ligands attached to the nanoparticles surface, said ligands containing at least one linking agent having a first portion linked to the nanoparticle and a second portion capable of linking to an affinity molecule,
- ligands attached to the nanoparticles surface for creating membrane crossing or cell-internalization capability,
- ligands attached to the nanoparticle surface for creating enzymatic functions,
- combination of the above.

16. An inorganic nanoparticle according to claim 14, wherein said nanoparticle is conjugated/linked to an affinity molecule capable of bonding with a detectable substance, wherein the affinity molecule is selected from a group consisting of:
- a biological material,
- an antibody,
- a nucleic acid,
- a protein,
- a polysaccharide,
- a sugar,
- a peptide, and
- a drug.

17. Method for obtaining a 3D reconstruction of a sample containing inorganic luminescent nanoparticles according to claim 1, comprising:
- obtaining a plurality of images of the sample, and
- removing one of more surface layers from the sample between the moment that the first image is obtained and the moment that the last image is obtained.

18. Method according to claim 17, in which the one or more surface layers are removed by etching.

19. Method according to claim 17, in which the one or more surface layers are removed by sublimation.

20. Method according to claim 17, in which the one or more surface layers are removed by sputtering.

21. Method according to any of claims 18-20, where the removal of the one or more surface layers is performed in-situ in a particle-optical apparatus.
